# EUROPÄISCHE PATENTANMELDUNG

(11) **EP 2 226 111 A1**
(43) Veröffentlichungstag der Anmeldung: **08.09.2010**
(21) Anmeldenummer: 10001603.9
(22) Anmeldetag: 17.02.2010
(51) Int. Cl.: B01D 53/04

(54) **Verfahren zum Entfernen von Schwefel aus einem Gas, insbesondere aus einem Biogas und Vorrichtung zur Durchführung des Verfahrens**

(30) Priorität: 18.02.2009 DE 102009009143
(71) Anmelder: Grimm Maschinenbau GmbH, 26689 Nordloh (DE)
(72) Erfinder: Beeken, Peter, 26655 Westerstede-Ocholt (DE); Grimm, Ernst, 26689 Nordloh-Apen (DE)
(74) Vertreter: Jabbusch, Matthias

(57) **Zusammenfassung**

Um Schwefel aus einem Gas, insbesondere aus einem Biogas zu entfernen, wird das Gas durch wenigstens eine Aktivkohleschüttung geführt. Die Aktivkohleschüttung wird in wenigstens zwei baugleiche Behälter aufgeteilt, welche hintereinander durch das Gas durchströmt werden. Der zuerst vom Gas durchströmte Behälter wird nach Zusetzen seiner Aktivkohle mit Schwefel entfernt und durch den vom Gas zweitdurchströmten Behälter ersetzt. Ein mit frischer Aktivkohle gefüllter Behälter wird als zweitdurchströmter Behälter vorgesehen.

## Beschreibung

Die Erfindung betrifft ein Verfahren zum Entfernen von Schwefel aus einem Gas, insbesondere aus einem Biogas, bei dem das Gas durch wenigstens eine Aktivkohleschüttung geführt wird. Die Erfindung betrifft des Weiteren eine Vorrichtung zum Entfernen von Schwefel aus einem Gas, insbesondere aus einem Biogas, zur Durchführung des vorgenannten Verfahrens, umfassend wenigstens eine Aktivkohleschüttung.

Gase werden häufig für eine energetische Verwertung eingesetzt. Aus ihnen kann Heizwärme gewonnen werden, sie können auch zum Betrieb von Turbinen oder Motoren eingesetzt werden. Wenn in einem derartigen Gas ein zu hoher Anteil an Schwefel enthalten ist, dann ist der ordnungsgemäße Betrieb der Verbrennungsmaschinen oder Heizungseinrichtungen gefährdet.

Neben natürlichen Gasreserven werden zum Betrieb von Turbinen bzw. Motoren auch Biogase verwendet. Diese werden aus pflanzlichen und anderen Rohstoffen gewonnen. Problematisch ist, dass Biogase gleichfalls einen Anteil von Schwefel enthalten, der ihrer energetischen Verwertung entgegensteht. Auch bei einer Einspeisung des Biogases in Gasversorgungsleitungen ist der Schwefel hinderlich, da er sich an Baugruppen sowohl der Verbrennungsmaschinen als auch der Gasleitungen absetzen kann.

Ein Heraustrennen des Schwefels insbesondere aus Biogas wird daher im Stand der Technik mit Hilfe einer Aktivkohleschüttung vorgenommen. Die Aktivkohleschüttung ist in einem Behälter angeordnet, durch den das zu reinigende Gas geführt wird. Die Aktivkohle löst den Schwefel aus dem Gas heraus, der Schwefel setzt sich an der Aktivkohle ab. Nach einer bestimmten Zeitdauer ist an der Aktivkohle so viel Schwefel angelagert, dass die Aktivkohle keinen weiteren Schwefel aufnehmen kann, also zugesetzt ist. Beim Stand der Technik wird dann der Behälter entleert. Dazu ist in der Behälterwandung ein Mannloch vorgesehen, durch den die Kohle entfernt werden kann. Dazu muss ein Mann mit einer Schaufel in das Innere des Behälters steigen, um die Kohle herauszuschippen. Diese Arbeit ist beschwerlich und gesundheitsgefährdend. Zudem ist die Biogasanlage während dieser Rüstarbeiten stillzulegen, wodurch sich ein Betriebsstillstand ergibt, der Kosten verursacht.

Der Erfindung liegt die Aufgabe zu Grunde, ein Verfahren der vorbezeichneten Gattung vorzuschlagen, mit dem ein Herauslösen von Schwefel aus einem Gas mit kürzeren Stillstandszeiten einer Gasanlage sowie mit besseren Bedingungen für das Bedienungspersonal möglich ist. Zudem soll eine Vorrichtung zur Durchführung des Verfahrens aufgezeigt werden.

Die verfahrensseitige Lösung dieser Aufgabe zeichnet sich erfindungsgemäß dadurch aus, dass die Aktivkohleschüttung in wenigstens zwei baugleiche Behälter aufgeteilt wird, welche hintereinander durch das Gas durchströmt werden, dass der zuerst vom Gas durchströmte Behälter nach Zusetzen seiner Aktivkohle mit Schwefel entfernt und durch den vom Gas zweitdurchströmten Behälter ersetzt wird und dass ein mit frischer Aktivkohle gefüllter Behälter als zweitdurchströmter Behälter vorgesehen wird.

Bei dem erfindungsgemäßen Verfahren wird somit eine Aufteilung der Aktivkohle vorgenommen. Die Aktivkohle wird auf mehrere Behälter aufgeteilt, welche einen Gasströmungsweg für das mit Schwefel belastete Gas ausbilden. Das Gas tritt nacheinander durch die Behälter hindurch, der Schwefel kann sich an den Aktivkohlepartikeln in den Behältern ansetzen. Aus dem letzten Behälter tritt das Gas dann vom Schwefel gereinigt aus.

Verfahrensgemäß ist vorgesehen, dass der zuerst vom Gas durchströmte Behälter nach einem Zusetzen entfernt wird. Damit wird eine Teilmenge der Aktivkohle von der Aktivkohleschüttung entfernt. Weitere Behälter, beispielsweise ein bisher an zweiter Stelle stehender Behälter und ein an dritter Stelle stehender Behälter, können nun nachrücken, indem sie die Plätze 1 und 2 in der Gasdurchströmungsreihenfolge einnehmen. Ein neuer dritter Behälter mit frischer Aktivkohle wird dann an die dritte Position gesetzt. Es ist somit nicht erforderlich, die Kohle als solche per Hand aus einem Behälter zu entfernen, vielmehr ist nur einer der Behälter wegzunehmen und durch einen neuen Behälter an anderer Stelle zu ersetzen. Diese Ersetzungszeit ist kurz bemessen, so dass ein Stillstand der Gasanlage gleichfalls nur kurz bemessen ist. Zudem ist es nicht erforderlich, dass eine Bedienungsperson sich in die Nähe der Aktivkohle begibt, insbesondere dann, wenn sie mit Schwefel zugesetzt ist. Das Herauslösen und Hinzufügen von Behältern mit Teilmengen der Aktivkohle kann auf maschinelle Weise erfolgen, auch das Herausnehmen der zugesetzten Aktivkohle aus einem Behälter. Dieser Behälter kann dann gleich wieder mit frischer Aktivkohle befüllt werden und an den Schlusspunkt des Gasführungsweges gesetzt werden. Es kann aber auch ein weiterer Behälter vorgehalten werden, der bereits mit frischer Kohle gefüllt auf seinen Einsatz wartet.

Nach einer ersten Weiterbildung der Erfindung ist vorgesehen, dass die Behälter übereinander angeordnet werden. Diese Anordnung hat den Vorteil, dass durch ein Anheben oberer Behälter der zuerst vom Gas durchströmte Behälter auf einfache Weise zur Seite herausgezogen werden kann, während anschließend die weiteren Behälter abgelassen werden und an oberster Position ein neuer Behälter ergänzt wird. Das Gas strömt dann von unten nach oben durch die Aktivkohle der einzelnen Behälter hindurch.

Nach einer Weiterbildung des erfindungsgemäßen Verfahrens ist noch vorgesehen, dass das Gas in den ersten Behälter mit einem Überdruck von etwa 0,4 bar eingeführt wird. Das Gas wird somit mit einem leichten Überdruck in den ersten Behälter und in die nachfolgenden Behälter geführt. Dadurch kann es durch eine eng gepackte Aktivkohleschüttung hindurchtreten, wobei aufgrund des Überdruckes eine gleichmäßige Verteilung des Gases beim Hindurchtritt durch die Aktivkohle gewährleistet ist. Dadurch wird vorteilhaft erreicht, dass die Aktivkohle gleichmäßig über den Querschnitt des Aktivkohlebehälters hinweg mit Schwefel belastet wird und dadurch vorteilhaft ein vorzeitiges Zusetzen eines Bereiches der Aktivkohle verhindert ist.

Zur vorrichtungsseitigen Lösung der Aufgabe, für die selbstständiger Schutz beansprucht wird, ist vorgesehen, dass die Aktivkohleschüttung in wenigstens zwei Behältern angeordnet ist, dass die Behälter gasleitend miteinander verbunden sind und mit Befestigungsmitteln miteinander verbunden sind.

Durch diese konstruktive Lösung sind zwei Aktivkohlemengen ausbildbar, die in voneinander getrennten Behältern angeordnet sind. Jede Aktivkohlemenge ist damit unabhängig von der anderen, so dass der erste Behälter mit zugesetzter Aktivkohle aus der Vorrichtung entfernt werden kann, während der zweite Behälter mit noch nicht zugesetzter Aktivkohle in der Vorrichtung verbleiben kann und dann die Position des ersten Behälters einnimmt. Beide Behälter sind mit Befestigungsmitteln miteinander verbunden, diese Befestigungsmittel können gelöst werden, um die Lageänderungen der Behälter vornehmen zu können. Wenn die Behälter aufeinander liegen, ist ihre Verbindung gasdicht auszubilden. Dazu sind vorzugsweise im Verbindungsbereich zweier Behälter Dichtungen angeordnet. Für das Verbinden der Behälter miteinander können übliche Befestigungsmittel, wie Bolzen oder Kniehebelspanner, vorgesehen sein.

Vorzugsweise ist vorgesehen, dass die Behälter aufeinander liegen. Die Dichtungen werden dann durch die oben liegenden Behälter belastet, so dass eine dichte Verbindung hergestellt ist. Zudem ist das verfahrensgemäße Bewegen der Behälter dadurch erleichtert. Die Behälter weisen an ihrer Außenseite vorzugsweise Beschläge für das Ansetzen eines Hubmittels auf. Dieses Hubmittel, beispielsweise ein Kran oder ein Gabelstapler, kann obere Behälter anheben, um den untersten Behälter zu entfernen und anschließend die oberen Behälter an die Stelle des untersten Behälters abzusetzen. Mit den gleichen Hubmitteln ist dann in oberer Anordnung ein neuer Behälter mit frischer Aktivkohle ergänzbar.

Nach einer nächsten Weiterbildung der Erfindung ist vorgesehen, dass die Behälter auf eine Plattform aufgestellt sind, welche eine Wanne hat, in die eine Zuleitung für das zu reinigende Gas einmündet. Das zu reinigende Gas wird somit nicht unmittelbar aus einer Zuleitung in den untersten Behälter eingeführt. Damit sind am unteren Behälter keine Zuleitungsanschlüsse vorzusehen, es ist lediglich ein Übertritt von Gas aus der Wanne in den ersten Behälter zu gewährleisten. Die Plattform, auf der die Behälter aufstehen, weist dazu die Wanne auf. In dieser kann Gas aufgenommen werden, da die Zuleitung für das zu reinigende Gas in die Wanne einmündet. Aus der Wanne tritt das Gas dann in den ersten Behälter ein, hier kann ein Übertritt über die gesamte Weite der Wanne vorgesehen sein. Damit ist ein großflächiger und gleich verteilter Eintritt des Gases in die Aktivkohle vorteilhaft gewährleistet. Dazu ist noch vorgesehen, dass die Zuleitung für das Gas in der Wannenwandung in die Wanne einmündet. Die Zuleitung ist somit nur bis zur Wannenwandung geführt, sie steht nicht noch ein Stück weit in das Innere der Wanne vor. Das Gas tritt aus der Zuleitung unmittelbar in die Wanne ein, aufgrund des verfahrensgemäß vorgesehenen leichten Überdrucks von etwa 0,4 bar verteilt sich das Gas gleichmäßig in der Wanne, bevor es in die Aktivkohleschüttung des ersten Behälters eintritt.

Zur weiteren Ausbildung der erfindungsgemäßen Vorrichtung ist noch vorgesehen, dass jeder Behälter mit einem Schwefelkonzentrationsmessgerät ausgerüstet ist. Mit diesem Messgerät wird die Konzentration des Schwefels im Gas, insbesondere im Biogas gemessen. Am obersten Behälter ist ablesbar, ob der gewünschte niedrige Anteil an Schwefel im Gas erreicht ist. Wenn die Reinigungsleistung der Aktivkohle abnimmt, ist dies ein Zeichen dafür, dass die Aktivkohle im untersten Behälter zugesetzt ist. Dann wird der unterste Behälter entfernt und an anderer Stelle wird ein neuer Behälter hinzugefügt.

Schließlich kann noch vorgesehen sein, dass zwei Aktivkohleschüttungen vorgesehen sind, welche in voneinander separate Behältergruppen mit Behältern jeweils aufgeteilt sind, wobei eine sich in beide Behältergruppen aufzweigende Zuleitung für das Gas vorgesehen ist, in die ein Umschalter für das wahlweise Zuführen des Gases in die eine oder in die andere Behältergruppe eingesetzt ist. Nach dieser Weiterbildung wird das zu reinigende Gas entweder in die eine oder in die andere Behältergruppe eingeleitet. Die Behältergruppe, in die das Gas gerade nicht eingeleitet wird, steht dann für eine Umrüstung der Behälterordnungen bereit. Vorteil dieser Ausgestaltung ist, dass das Gas ohne Unterbrechung fließen kann, es ist lediglich an die eine oder andere Behältergruppe zu leiten. Stillstandszeiten der Gasanlage, beispielsweise einer Biogasanlage, sind praktisch ausgeschlossen.

Ein Ausführungsbeispiel der erfindungsgemäßen Vorrichtung, aus dem sich weitere erfinderische Merkmale ergeben, ist in der Zeichnung dargestellt. Es zeigen:
- Fig. 1:: eine Seitenansicht einer Vorrichtung mit drei übereinander angeordneten Behältern,
- Fig. 2:: eine Ansicht der Vorrichtung gemäß Fig. 1 von oben,
- Fig. 3:: eine Ansicht der einzelnen Bauelemente und Behälter der Vorrichtung gemäß Fig. 1 und
- Fig. 4:: eine Ansicht eines die Vorrichtung gemäß Fig. 1 während der Behälter-Wechselphase ersetzenden Zusatzbehälters.

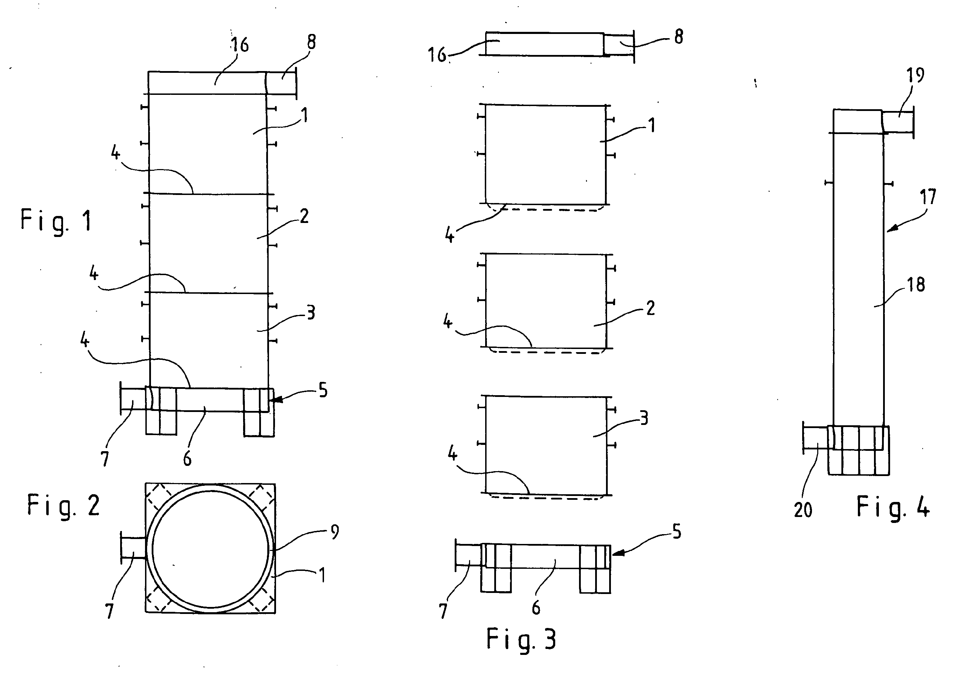

In Fig. 1 ist eine Seitenansicht einer Ausführung einer Vorrichtung zum Entfernen von Schwefel aus einem Gas, insbesondere aus einem Biogas, das durch wenigstens eine Aktivkohleschüttung geführt wird, dargestellt.

Die Vorrichtung besteht bei dieser Ausführung aus drei übereinander angeordneten, baugleichen Behältern 1, 2 und 3. Jeder Behälter 1, 2 und 3 enthält eine Aktivkohleschüttung. Die Behälter sind gasleitend untereinander verbunden. Jeder Behälter 1, 2, 3 weist einen Boden 4 auf, der als Siebblech ausgebildet ist. Dadurch sind die gemäß Fig. 1 aufeinander gestellten Behälter 1, 2 und 3 gasleitend miteinander verbunden.

Der untere Behälter 3 ist auf eine Plattform 5 gestellt, die eine Wanne 6 aufweist. In die Wanne 6 mündet die Zuleitung 7 für ein in die aus übereinander gesetzten Behältern gebildete Säule einzuleitendes Gas.

Im gegenseitigen Verbindungs- bzw. Anlagebereich zueinander benachbarter, aufeinander stehender Behälter 1, 2 und 3 sind Dichtungen angeordnet, die z. B. als Gummidichtungen ausgebildet sein können.

Der in einer aus aufeinander gestellten Behältern gebildeten Säule oberste Behälter 1 ist mit einem Deckelteil 16 verschlossen, welches, ebenso wie die untere Plattform 5, eine Wanne aufweist. Die Wanne ist ebenfalls mit einem Stutzen 8 ausgerüstet, über den Gas geleitet werden kann.

In Fig. 2 ist eine Draufsicht der Vorrichtung gemäß Fig. 1 dargestellt, bei der jedoch das obere Deckelteil 16 abgenommen ist. Die dadurch sichtbare freie Oberfläche der Aktivkohlebefüllung 9 des Behälters 1 ist punktiert dargestellt.

In Fig. 3 sind die Behälter 1, 2 und 3 der Vorrichtung gemäß Fig. 1 jeweils einzeln gezeichnet. Auch die Plattform 5 mit der Wanne 6 und der Zuleitung 7 ist in Fig. 3 ebenso gezeichnet, wie das Deckelteil 16 mit seinem Stutzen 8.

In Fig. 4 ist eine Seitenansicht eines die Vorrichtung gemäß Fig. 1 während der Phase der Auswechslung eines oder mehrerer ihrer Behälter 1, 2 oder 3 ersetzenden Zusatzbehälters 17 dargestellt.

Der Zusatzbehälter 17 ist, ebenso wie die Vorrichtung, als eine stehende Säule 18 ausgebildet, die über Anschlüsse 19 und 20 mit Stutzen für eine Zu- und Ableitung von Gas verfügt.

Wird die Säule 18 während der Zeit der Auswechslung eines der Behälter 1, 2 und 3 in den Gasförderweg, der der Vorrichtung gemäß Fig. 1 zugeordnet ist, eingeschaltet, können die Förderanlagen für Gas unverändert weiterlaufen. Die Strömungswege müssen nicht abgeschaltet bzw. unterbrochen werden, denn der Gasförderweg läuft unverändert über die zusätzliche Säule 18.

Nach der Beendigung des Auswechselns einer der Behälter 1, 2 und/oder 3 kann die Vorrichtung wieder dem Gasförderweg durch die Vorrichtung gemäß Fig. 1 zugeschaltet werden, so dass die zusätzliche Säule 18 dann wieder stilllegbar ist.

## Patentansprüche

1. Verfahren zum Entfernen von Schwefel aus einem Gas, insbesondere aus einem Biogas, bei dem das Gas durch wenigstens eine Aktivkohleschüttung geführt wird,
**dadurch gekennzeichnet,**
**dass** die Aktivkohleschüttung (9) in wenigstens zwei baugleiche Behälter (1, 2, 3) aufgeteilt wird, welche hintereinander durch das Gas durchströmt werden, dass der zuerst vom Gas durchströmte Behälter (3) nach Zusetzen seiner Aktivkohle mit Schwefel entfernt und durch den vom Gas zweitdurchströmten Behälter (2) ersetzt wird und dass ein mit frischer Aktivkohle gefüllter Behälter als zweitdurchströmte Behälter vorgesehen wird.

2. Verfahren nach Anspruch 1, **dadurch gekennzeichnet, dass** die Behälter (1, 2, 3) übereinander angeordnet werden.

3. Verfahren nach Anspruch 1 oder 2, **dadurch gekennzeichnet, dass** das Gas in den ersten Behälter (3) mit einem Überdruck von etwa 0,4 bar eingeführt wird.

4. Vorrichtung zum Entfernen von Schwefel aus einem Gas, insbesondere aus einem Biogas, zur Durchführung des Verfahrens nach einem der Ansprüche 1 bis 3, umfassend wenigstens eine Aktivkohleschüttung,
**dadurch gekennzeichnet,**
**dass** die Aktivkohleschüttung in wenigstens zwei Behältern (1, 2) angeordnet ist, dass die Behälter (1, 2, 3) gasleitend miteinander verbunden sind und mit Befestigungsmitteln miteinander verbunden sind.

5. Vorrichtung nach Anspruch 4, **dadurch gekennzeichnet, dass** im Verbindungsbereich zweier Behälter (1, 2, 3) Dichtungen angeordnet sind.

6. Vorrichtung nach Anspruch 4 oder 5, **dadurch gekennzeichnet, dass** die Verbindungsmittel zum Verbinden zweier Behälter (1, 2, 3) als Kniehebelspanner ausgebildet sind.

7. Vorrichtung nach einem der Ansprüche 4 bis 6, **dadurch gekennzeichnet, dass** die Behälter (1, 2, 3) aufeinander liegen.

8. Vorrichtung nach Anspruch 7, **dadurch gekennzeichnet, dass** die Behälter auf eine Plattform (5) aufgestellt sind, welche eine Wanne (6) hat, in die eine Zuleitung (7) für das zu reinigende Gas einmündet.

9. Vorrichtung nach Anspruch 8, **dadurch gekennzeichnet, dass** die Zuleitung für das Gas in der Wannenwandung in die Wanne (6) einmündet.

10. Vorrichtung nach einem der Ansprüche 4 bis 9, **dadurch gekennzeichnet, dass** jeder Behälter (1, 2, 3) mit einem Schwefelkonzentrationsmessgerät ausgerüstet ist.

11. Vorrichtung nach einem der Ansprüche 4 bis 10, **dadurch gekennzeichnet, dass** an jedem Behälter (1, 2, 3) Beschläge für das Ansetzen eines Hubmittels angeordnet sind.

12. Vorrichtung nach einem der Ansprüche 4 bis 11, **dadurch gekennzeichnet, dass** zwei Aktivkohleschüttungen vorgesehen sind, welche in voneinander separate Behältergruppen mit Behältern jeweils aufgeteilt sind, wobei eine sich in beide Behältergruppen sich aufzweigende Zuleitung für das Gas vorgesehen ist, in die ein Umschalter für das wahlweise Zuführen des Gases in die eine oder in die andere Behältergruppe eingesetzt ist.
